(19) **Europäisches Patentamt**
**European Patent Office**
**Office européen des brevets**

(11) **EP 3 344 317 B1**

(12) **EUROPÄISCHE PATENTSCHRIFT**

(45) Veröffentlichungstag und Bekanntmachung des Hinweises auf die Patenterteilung:
**28.10.2020 Patentblatt 2020/44**

(21) Anmeldenummer: **16758203.0**

(22) Anmeldetag: **01.09.2016**

(51) Int Cl.:
***A61M 16/04*** *(2006.01)* ***A61M 16/16*** *(2006.01)*
***A61M 16/20*** *(2006.01)* ***A61M 16/00*** *(2006.01)*

(86) Internationale Anmeldenummer:
**PCT/EP2016/070572**

(87) Internationale Veröffentlichungsnummer:
**WO 2017/037152 (09.03.2017 Gazette 2017/10)**

(54) **BEATMUNGSVORRICHTUNG MIT FEHLERERFASSUNG FÜR DURCHFLUSSSENSOREN**

VENTILATOR WITH ERROR DETECTION FOR FLOW SENSORS

DISPOSITIF RESPIRATOIRE À DÉTECTION D'ERREUR POUR CAPTEURS DE DÉBIT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.09.2015 DE 102015216895**

(43) Veröffentlichungstag der Anmeldung:
**11.07.2018 Patentblatt 2018/28**

(73) Patentinhaber: **Hamilton Medical AG**
**7402 Bonaduz (CH)**

(72) Erfinder:
• **SCHRANZ, Christoph**
**7402 Bonaduz (CH)**

• **FRANKE, Karolin**
**8003 Zürich (CH)**
• **NOVOTNI, Dominik**
**7000 Chur (CH)**

(74) Vertreter: **Ruttensperger Lachnit Trossin Gomoll Patent- und Rechtsanwälte PartG mbB Postfach 20 16 55 80016 München (DE)**

(56) Entgegenhaltungen:
**WO-A1-03/055552**

**Beschreibung**

Die vorliegende Erfindung betrifft eine Beatmungsvorrichtung zur künstlichen Beatmung gemäß dem Oberbegriff des Anspruchs 1.

Eine gattungsgemäße Beatmungsvorrichtung ist aus der WO 03/055552 A1 bekannt. Diese offenbart die Ermittlung von Leckagen an einer Inspirationsleitung durch Vergleich von Erfassungswerten unterschiedlicher Sensoren, die den Inspirationsstrom erfassen. Die WO 03/055552 A1 lehrt weiter, die Plausibilität eines Sensormesswerts durch Überprüfung eines von demselben Sensor gelieferten Gradienten oder des Messwert-Vorzeichens zu prüfen.

Eine weitere Beatmungsvorrichtung ist beispielsweise im Markt als Produkt "SERVO-U" der Firma Maquet bekannt. Diese bekannte Beatmungsvorrichtung verwendet einen internen Sensor im Inneren eines Beatmungsgeräts als distalen Durchflusssensor. An dem Beatmungsgerät ist das distale Ende der Beatmungsleitungsanordnung angeschlossen. Weiter verwendet die bekannte Beatmungsvorrichtung einen proximalen Durchflusssensor in Form eines Heißdrahtanemometers in einem Y-Verbindungsstück, in welchem in Richtung zum Patienten hin ein inspiratorischer und ein expiratorischer Beatmungsschlauch zu einer gemeinsamen zum Patienten hin führenden Beatmungsleitung vereinigt werden. Die Bedienungsanleitung zu dieser bekannten Beatmungsvorrichtung gibt an, dass die Ausgaben von internen Druck- und Flusssensoren mit dem Messergebnis des proximalen Sensors in dem Y-Verbindungsstück verglichen werden und der proximale Sensor deaktiviert wird, wenn zwischen den zum Vergleich herangezogenen Werten eine signifikante Abweichung festgestellt wird.

Nachteilig an der bekannten Beatmungsvorrichtung und der dort offenbarten Fehlererfassung ist, dass die bloße Fehlererfassung aufgrund eines Vergleichs von Messwerten zweier unterschiedlicher Sensoren nur einen Teil der tatsächlich an einer Sensoranordnung auftretenden Fehler zu erfassen vermag. Außerdem liegt zwischen den beiden Sensoren die Beatmungsleitungsanordnung in Form eines elastischen Schlauches, der bei jedem Atemhub von dem ihn durchströmenden Atemgas gegen seine Elastizität gedehnt wird. Aufgrund dieser Schlauchelastizität liefern die beiden an unterschiedlichen Stellen im Atemgasstrom angeordneten Durchflusssensoren selbst bei gleichem effektiven Atemgasstrom an beiden Erfassungsstellen unterschiedliche Messwerte. Das den Schlauch dehnende Atemgas passiert nämlich den distalen Durchflusssensor, jedoch nicht den proximalen Durchflusssensor, weshalb die Messwerte des distalen Durchflusssensors in der Regel höher sind als jene des proximalen.

Beatmungsvorrichtungen sind für Patienten, die selbstständig nicht oder nicht in ausreichendem Maße atmen können, lebenswichtige Geräte. Ihre korrekte Funktion ist daher von entscheidender Bedeutung. Die korrekte Funktion der Beatmungsvorrichtungen hängt wiederum von der möglichst präzisen Erfassung der einem Patienten zugeführten Atemgasmenge ab.

Es ist daher Aufgabe der vorliegenden Erfindung, eine Beatmungsvorrichtung der eingangs genannten Art derart weiterzubilden, dass etwaige Fehlfunktionen der Durchflusssensoranordnung frühzeitig zuverlässig erkannt werden können.

Diese Aufgabe wird erfindungsgemäß gelöst durch eine gattungsgemäße Beatmungsvorrichtung mit allen Merkmalen des Anspruchs 1. Durch die Verwendung von Änderungswerten des Messsignals anstelle des Messsignals selbst können Fehler der Durchflusssensoranordnung früher oder/und zuverlässiger als bis her erfasst werden. Somit ist es beispielsweise möglich, einen Fehler bereits zu erfassen, wenn sich die Messwerte des proximalen und des distalen Durchflusssensors zwar nur sehr geringfügig, jedoch in unterschiedliche Richtungen ändern, etwa der eine Durchflusssensor eine Erhöhung der Durchflussmenge anzeigt und der jeweils andere Durchflusssensor eine Verringerung. Somit kann bereits auf einen Fehler geschlossen werden, obwohl ein Vergleich der Messwerte der beiden Durchflusssensoren miteinander noch keine signifikante Abweichung voneinander aufzeigen würde.

Alternativ oder zusätzlich kann der Änderungswert des Messsignals des einen Durchflusssensors aus proximalem und distalem Durchflusssensor mit einem Messsignal desselben Durchflusssensors - also wiederum des einen Durchflusssensors - verglichen werden, sodass allein aufgrund von Signalen ein und desselben Durchflusssensors: proximale oder distale Durchflusssensor, ohne Berücksichtigung der Signale des jeweils anderen Durchflusssensors auf einen Fehler des einen Durchflusssensors geschlossen werden kann, etwa wenn dessen Änderungswert bezogen auf das letzte Messsignal vor der Änderung aufgrund der vorherrschenden Betriebsbedingungen ungewöhnlich groß oder ungewöhnlich klein ist.

Wenn dabei Änderungswerte unterschiedlicher Sensoren miteinander verglichen werden, handelt es sich dabei um gleichzeitige oder quasi-gleichzeitige Änderungswerte, also um Änderungswerte die zum selben Zeitpunkt oder am selben Atemhub ermittelt werden.

Wird ein Änderungswert eines Messsignals mit einem Messsignal verglichen, handelt es sich dabei um einen Vergleich eines Änderungswerts mit einem zeitlich früher erfassten Messsignal, vorzugsweise mit einem zeitlich unmittelbar vor dem Änderungswert ermittelten Messsignal.

Eine besonders sichere Fehlererkennung kann dabei erhalten werden, wenn die obigen Möglichkeiten zur Erkennung von Fehlern der Durchflusssensoranordnung nicht nur alternativ, sondern kumulativ angewendet werden.

Wie oben bereits angedeutet wurde, ist eine schnelle und effektive Fehlererkennung dann möglich, wenn die Steuereinrichtung dazu ausgebildet ist, dann auf einen Fehler der Durchflusssensoranordnung zu schließen, wenn der Änderungswert des Messsignals des einen Durchflusssensors aus distalem und proximalem Durchflusssensor eine entgegengesetzte Änderungsrichtung aufweist als der Änderungswert des jeweils anderen Durchflusssensors. Hierzu müssen nicht einmal Zahlenwerte miteinander verglichen werden, sondern es kann ein Vergleich von Vorzeichen der jeweiligen Änderungswerte miteinander ausreichen, um auf einen Fehler zu schließen.

An der Durchflusssensoranordnung, insbesondere am proximalen Durchflusssensor, treten nach bisherigen Erfahrungen im Wesentlichen zwei unterschiedliche Fehlerarten auf, die sich hauptsächlich durch die Zeitdauer unterscheiden, in welcher sie sich entwickeln und auf die Beatmungsvorrichtung auswirken.

Der eine Fehler, der nachfolgend als "Sprungfehler" bezeichnet wird, tritt plötzlich auf und führt innerhalb weniger Sekunden zu einem beträchtlichen Fehler im Messergebnis.

Der andere Fehler, der nachfolgend als "Driftfehler" bezeichnet wird, tritt schleichend auf und entwickelt sich über mehrere Minuten, bis hin zu einer halben Stunde oder einer Dreiviertelstunde, zu einem beträchtlichen Fehler im Messergebnis.

Nach bisherigen Erkenntnissen stehen diese beiden Fehler höchstwahrscheinlich mit dem Niederschlag von Kondensat im proximalen Durchflusssensor im Zusammenhang. Dadurch, dass der proximale Durchflusssensor sehr nahe am Patienten vorgesehen ist, kann dieser stärker durch die Feuchtigkeit in der Atemluft beeinflusst werden. Während der Sprungfehler vor allem bei Durchflusssensoren, die nach dem Differenzdruckprinzip arbeiten, sehr gut verstanden wird, konnte für den Driftfehler bisher lediglich phänomenologisch festgestellt werden, dass dieser verschwindet, wenn man einen mit Kondensat beschlagenen Durchflusssensor durch einen trockenen ersetzt hat, nachdem man den Driftfehler erkannt hat.

Wenngleich wenigstens der proximale Durchflusssensor bevorzugt ein nach dem Differenzdruckprinzip arbeitender Durchflusssensor ist, wie er etwa aus der DE 10 2010 040287 A1 bekannt ist, soll die vorliegende Erfindung nicht auf Beatmungsvorrichtungen mit einer Durchflusssensoranordnung mit wenigstens einem nach dem Differenzdruckprinzip arbeitenden Durchflusssensor beschränkt sein. Die vorliegend vorgeschlagene verbesserte Beatmungsvorrichtung mit erhöhter Genauigkeit in der Fehlererkennung bei Durchflusssensoren kann grundsätzlich jeden beliebigen Durchflusssensor nutzen, unabhängig davon, welche physikalischen Wirkprinzipien er zur Messung des Gasflusses verwendet.

Der Vollständigkeit halber sei erwähnt, dass nach derzeitiger Theorie der Sprungfehler in einem nach dem Differenzdruckprinzip arbeitenden proximalen Durchflusssensor gemäß der DE 10 2010 040287 A1 dann auftritt, wenn eine bewegliche Klappe, welche zwei im Sensor in Durchströmungsrichtung aufeinander folgend angeordnete Druckmesskammern trennt, in im Sensor angesammeltes Kondensat eintaucht und dadurch für ihre Bewegung einen größeren Widerstand zu überwinden hat als in einem trockenen Sensor.

Der Sprungfehler kann erfindungsgemäß dann schnell und sicher erkannt werden, wenn die Steuereinrichtung dazu ausgebildet ist, dann auf einen Fehler der Durchflusssensoranordnung zu schließen, wenn

- ein Verhältnis eines Summenwerts von nacheinander anfallenden Änderungswerten des Messsignals des proximalen Durchflusssensors zu einer ersten Bezugsgröße einen vorbestimmten Bezugsschwellenwert übersteigt und
- wenn ein Verhältnis eines Summenwerts von nacheinander anfallenden Änderungswerten des Messsignals des distalen Durchflusssensors zu einer zweiten Bezugsgröße einen vorbestimmten Bezugsschwellenwert unterschreitet.

Dabei werden also nacheinander anfallende Änderungswerte des Messsignals des proximalen und des distalen Durchflusssensors aufsummiert, um die über die Dauer der Summierung insgesamt auftretende Änderung des Messsignals des jeweiligen Durchflusssensors zu quantifizieren. Dieser Summenwert der Änderung des Messsignals kann dann mit einer Bezugsgröße in Bezug gesetzt werden, um zu beurteilen, ob die Durchflusssensoranordnung korrekt arbeitet oder nicht. Die Beurteilung erfolgt anhand eines vorbestimmten Bezugsschwellenwertes.

Bevorzugt werden zur Bildung des Summenwerts unmittelbar nacheinander anfallende Änderungswerte von Messsignalen aufsummiert, um den Summenwert über einen möglichst kurzen Zeitraum bilden zu können und so schnell einen entsprechenden Fehler in der Durchflusssensoranordnung ermitteln zu können. Außerdem kann so sichergestellt werden, dass kein Änderungswert eines Messsignals für die Beurteilung der Korrektheit des Betriebs der Durchflusssensoranordnung außer Betracht bleibt.

Gemäß einer bevorzugten Weiterbildung der vorliegenden Erfindung ist die erste oder/und die zweite Bezugsgröße ein Messsignalwert des proximalen Durchflusssensors. Dadurch können die Änderungswerte des proximalen Durchflusssensors und die Änderungswerte des distalen Durchflusssensors jeweils mit einer Summe der Änderungen des proximalen Durchflusssensors als dem fehleranfälligeren Durchflusssensor aus proximalem und distalem Durchflusssensor in Bezug gesetzt werden. Dies ist für die Beurteilung des korrekten Betriebs der Durchflusssensoranordnung häufig hilfreich, da der absolute Wert einer Änderung eines Messsignals hinsichtlich seiner Plausibilität in der Regel nur im Zusammenhang mit dem Messwert, der durch die Änderung geändert wird, sinnvoll beurteilbar ist. Dementsprechend kann die Genauigkeit der Fehlererfassung, insbesondere eines Sprungfehlers, dadurch erhöht werden, dass die erste oder/und die zweite Bezugsgröße ein Messsignalwert des proximalen Durchflusssensors bei oder unmittelbar vor dem Beginn der Aufsummierung der Änderungswerte ist. Dann stehen die Änderungen in unmittelbarem Bezug zu dem Messsignal, mit dem sie zur Beurteilung ihrer Plausibilität bzw. Richtigkeit in Beziehung gesetzt werden.

Die Beatmungsvorrichtung muss jedoch nicht dauerhaft eine Fehlererfassung während ihres gesamten Betriebs durchführen. Es genügt, Rechenleistung der Steuereinrichtung der Beatmungsvorrichtung dann für eine genauere Fehlererfassung bereitzustellen, wenn sich diese Notwendigkeit aus dem Betrieb der Beatmungsvorrichtung heraus abzeichnet.

Bevorzugt ist daher die Steuereinrichtung der Beatmungsvorrichtung dazu ausgebildet, den Betrieb der Durchflusssensoranordnung dauerhaft gemäß einem ersten, weniger Rechenleistung erfordernden Prüfverfahren auf Fehler zu überwachen, und ist weiter dazu ausgebildet, dann, wenn das erste Prüfverfahren einen Fehlerverdachtszustand der Durchflusssensoranordnung aufzeigt, den Betrieb der Durchflusssensoranordnung gemäß einem zweiten, mehr Rechenleistung erfordernden Prüfverfahren auf Fehler zu überwachen.

Für den oben bereits beschriebenen bevorzugten Fall eines intensiven Prüfverfahrens kann dies bedeuten, dass die Steuereinrichtung dazu ausgebildet ist, mit der Aufsummierung von Änderungswerten zu beginnen, wenn der Messsignalwert oder/und der Änderungswert des proximalen Durchflusssensors einen vorbestimmten Signalschwellenwert übersteigt. Die Prüfung, ob der Messsignalwert oder/und der Änderungswert des proximalen Durchflusssensors einen vorbestimmten Signalschwellenwert übersteigt, wird bevorzugt dauerhaft ausgeführt und entspricht dem oben abstrakt bezeichneten ersten Prüfverfahren, welches weniger Rechenleistung erfordert. Dann, wenn der Änderungswert oder/und der Messsignalwert des proximalen Durchflusssensors den vorbestimmten Signalschwellenwert übersteigt, ist ein Fehlerverdachtszustand ermittelt, sodass bevorzugt mit der oben beschriebenen Aufsummierung von Änderungswerten und deren Vergleich mit Bezugswerten begonnen wird.

Parallel kann das erste Prüfverfahren weitergeführt werden und es kann daran gedacht sein, das zweite Prüfverfahren wieder zu beenden, wenn das erste Prüfverfahren über eine vorbestimmte Anzahl von Atemzyklen keinen Fehlerverdachtszustand mehr aufzeigt, also beispielsweise wenn der Messsignalwert oder/und der Änderungswert des proximalen Durchflusssensors über eine vorbestimmte Anzahl nacheinander ausgewerteter Messsignale den vorbestimmten Signalschwellenwert nicht mehr übersteigt.

Es kann jedoch ebenso daran gedacht sein, das zweite, mehr Rechenleistung erfordernde Prüfverfahren bis zum Ende des Betriebs der Beatmungsvorrichtung fortzusetzen, wenn einmal ein Fehlerverdachtszustand ermittelt wurde.

In einer bevorzugten Ausgestaltung der Beatmungsvorrichtung kann der distale Durchflusssensor zur möglichst genauen Regelung des Atemgasflusses während eines Atemhubs dienen. Dies bedeutet, dass der Atemgasfluss während eines Atemhubs auf Grundlage des vom distalen Durchflusssensor gelieferten Messsignals verändert wird.

Demgegenüber kann der proximale Durchflusssensor zur Regelung des Minutenvolumens herangezogen werden, wie es etwa bei den bekannten Beatmungsmodi ASV, Intellivent-ASV und APV verwendet wird. Hierbei wird bevorzugt das Minutenvolumen nur atemhubsweise eingestellt bzw. geregelt, d. h. es erfolgt keine Veränderung des Atemgasflusses während des Atemhubs auf Grundlage des Messsignals des proximalen Durchflusssensors. Dies hat den Vorteil, dass das dem Patienten tatsächlich verabreichte Atemgasvolumen mit dem näher beim Patienten angeordneten proximalen Durchflusssensor genauer als mit dem distalen Durchflusssensor bestimmt werden kann, unter anderem auch weil die Genauigkeit des Messsignals negativ beeinflussende Effekte, wie etwa die Elastizität von Schlauchleitungen im Beatmungsschlauch, sich auf das Messsignal des proximalen Durchflusssensors nicht oder nur in sehr geringem Umfang auswirken.

Aufgrund der Anordnung des distalen und des proximalen Durchflusssensors wird der distale Durchflusssensor bevorzugt ausschließlich mit inspiratorischem Atemgas durchströmt, während der proximale Durchflusssensor sowohl mit inspiratorischem als auch mit exspiratorischem Atemgas durchströmt wird. Auch hieraus ergibt sich eine verglichen mit jener des distalen Durchflusssensors erhöhte Fehleranfälligkeit des proximalen Durchflusssensors.

Da die Beatmungsvorrichtung, wenngleich sie wie oben beschrieben Atemgasflüsse auch während eines Atemhubs verändert, im Wesentlichen den Patienten mit diskreten Atemhüben beatmet, kann bevorzugt daran gedacht sein, dass auch die Steuereinrichtung dazu ausgebildet ist, die Messsignale des proximalen und des distalen Durchflusssensors atemhubsweise zu verarbeiten. Dies hat den Vorteil, dass die meisten Beatmungsparameter, welche den Betrieb der Beatmungsvorrichtung bestimmen, atemhubsweise, also auf den jeweiligen auszuführenden Atemhubs bezogen, vorliegen.

In diesem Fall kann gemäß einer bevorzugten Weiterbildung zur weiteren Erhöhung der Genauigkeit der Fehlererkennung daran gedacht sein, dann, wenn der Messsignalwert des proximalen Durchflusssensors den vorbestimmten Signalschwellenwert bei einem Schwellen-Atemhub übersteigt, die erste oder/und die zweite Bezugsgröße der Messsignalwert des proximalen Durchflusssensors ist, welcher dem dem Schwellen-Atemhub unmittelbar vorhergehenden Atemhub zugeordnet ist. Auch dies dient wiederum der Herstellung eines möglichst unmittelbaren zeitlichen Zusammenhangs zwischen den für die Fehlererkennung der Durchflusssensoranordnung heranzuziehenden Messsignalen oder/und Änderungswerten und der Bezugsgröße, mit der die Signale bzw. Werte in Beziehung gesetzt werden.

Versuche haben gezeigt, dass der Driftfehler dadurch gut erfasst werden kann, dass die Steuereinrichtung dazu ausgebildet ist, dann auf einen Fehler der Durchflusssensoranordnung zu schließen, wenn

- ein Verhältnis eines Summenwerts von nacheinander anfallenden, vorzugsweise unmittelbar aufeinanderfolgenden, Änderungswerten des Messsignals des proximalen Durchflusssensors zu einer dritten Bezugsgröße einen vorbestimmten Bezugsschwellenwert übersteigt und
- wenn ein Verhältnis eines Summenwerts von nacheinander anfallenden, vorzugsweise unmittelbar aufeinanderfolgenden, Änderungswerten des Messsignals des distalen Durchflusssensors zu einer vierten Bezugsgröße einen vorbestimmten Bezugsschwellenwert unterschreitet,

oder dann auf einen Fehler der Durchflusssensoranordnung zu schließen, wenn

- ein Verhältnis eines Summenwerts von nacheinander anfallenden, vorzugsweise unmittelbar aufeinanderfolgenden, Änderungswerten des Messsignals des proximalen Durchflusssensors zu einer fünften Bezugsgröße einen vorbestimmten Bezugsschwellenwert unterschreitet und
- wenn ein Verhältnis eines Summenwerts von nacheinander anfallenden, vorzugsweise unmittelbar aufeinanderfolgenden, Änderungswerten des Messsignals des distalen Durchflusssensors zu einer sechsten Bezugsgröße einen vorbestimmten Bezugsschwellenwert übersteigt.

Es sei an dieser Stelle klargestellt, dass die Verwendung von Ordinalzahlen zur Unterscheidung der Bezugsschwellenwerte in der vorliegenden Anmeldung die Reihenfolge der Nennung der Bezugsschwellenwerte im Anmeldungstext wiedergibt und nicht als Angabe einer Mindestanzahl an Bezugsschwellenwerten in der Beatmungsvorrichtung verstanden werden soll.

Zu den Vorteilen der Verwendung unmittelbar aufeinanderfolgender Änderungswerte sei auf die obigen Ausführungen zur Erfassung des Sprungfehlers verwiesen, die auch hier gelten. Die genannten Bezugsschwellenwerte lassen sich durch Versuche unter Berücksichtigung medizinischer Randbedingungen ohne nennenswerten Aufwand ermitteln.

Grundsätzlich kann der Driftfehler mit den vorstehend genannten Bedingungen bereits zuverlässig ermittelt werden. Eine noch höhere Genauigkeit der Erfassung des Driftfehlers kann dadurch erreicht werden, dass die Steuereinrichtung dazu ausgebildet ist, nur dann auf einen Fehler der Durchflusssensoranordnung zu schließen, wenn zusätzlich

- der Summenwert von nacheinander anfallenden, vorzugsweise unmittelbar aufeinanderfolgenden, Änderungswerten des Messsignals des proximalen Durchflusssensors einen vorbestimmten ersten Summenschwellenwert übersteigt,
  oder/und
- der Summenwert von nacheinander anfallenden, vorzugsweise unmittelbar aufeinanderfolgenden, Änderungswerten des Messsignals des distalen Durchflusssensors einen vorbestimmten zweiten Summenschwellenwert übersteigt.

Wiederum erhöht die kumulative Anwendung obiger Bedingungen die Genauigkeit. Die Aufsummierung erfolgt für beide Sensoren bevorzugt von einem gemeinsamen Beginn bis zum jeweils aktuellen Atemhub.

Grundsätzlich können beliebige geeignet erscheinende Bezugsgrößen für die Beurteilung der Plausibilität bzw. Korrektheit von Änderungswerten des Messsignals eines der beiden oder beider Durchflusssensoren herangezogen werden. Wie oben bereits dargelegt wurde, ist eine besonders aussagekräftige Bezugsgröße zur Bewertung von Änderungen der Messsignale oder von den Messsignalen selbst, ein Messsignalwert des proximalen Durchflusssensors. Daher kann gemäß einer vorteilhaften Weiterbildung der vorliegenden Erfindung vorgesehen sein, dass die dritte oder/und die fünfte Bezugsgröße ein Messsignalwert des proximalen Durchflusssensors ist. Um einen möglichst unmittelbaren zeitlichen Zusammenhang zwischen Bezugsgröße und zu beurteilenden Größen herzustellen, ist die dritte oder/und die fünfte Bezugsgröße bevorzugt ein Messsignalwert des proximalen Durchflusssensors bei oder unmittelbar vor dem Beginn der Aufsummierung der Änderungswerte.

Mit der gleichen Motivation ist bei einer bevorzugten Weiterbildung der vorliegenden Erfindung vorgesehen, dass die vierte oder/und die sechste Bezugsgröße ein Messsignalwert des distalen Durchflusssensors ist, insbesondere ein Messsignalwert des distalen Durchflusssensors bei oder unmittelbar vor dem Beginn der Aufsummierung der Änderungswerte ist.

Auch hier kann vorgesehen sein, dass die Aufsummierung von Änderungswerten zur Bildung von Summenwerten beginnt, wenn der Betrag des Änderungswerts des Messsignals des proximalen oder/und des distalen Durchflusssensors einen vorbestimmten Betragsschwellenwert übersteigt.

In diesem Falle ist wiederum der Vergleich des Betrags des Änderungswerts des Messsignals des proximalen oder/und des distalen Durchflusssensors mit einem vorbestimmten Betragsschwellenwert ein erstes, weniger Rechenleistung erforderndes Prüfverfahren im obigen Sinne, ausgehend von welchem das oben beschriebene zweite, mehr Rechenleistung erfordernde Prüfverfahren ausgelöst werden kann. Hinsichtlich der Dauer der Ausführung des mehr Rechenleistung erfordernden Prüfverfahrens zur Feststellung des Driftfehlers wird auf die obigen Ausführungen zur Ermittlung eines Sprungfehlers verwiesen, die auch vorliegend im Zusammenhang mit der Ermittlung des Driftfehlers gelten.

Ein erstes Prüfverfahren zur Bestimmung, ob ein zweites, mehr Rechenleistung erforderndes Prüfverfahren gestartet werden soll, kann alternativ oder zusätzlich ein Vergleich der Änderungsrichtung, also etwa der Vorzeichen, von Änderungswerten des proximalen und des distalen Durchflusssensors auf jeweils denselben Atemhub bezogen sein. Sind die Vorzeichen verschieden, ändern sich also die Messsignale von proximalem und distalem Durchflusssensor für denselben Atemhub in unterschiedliche Richtungen, dann liegt ein ausreichender Fehlerverdacht vor, um das zweite Prüfverfahren zu beginnen.

Um Störeffekte möglichst nicht in die Berücksichtigung der zur Fehlererkennung herangezogenen Größen einzubeziehen, kann die Steuereinrichtung dazu ausgebildet sein, die Messsignale der Durchflusssensoren durch Filterung zu glätten. Ein solcher Störeffekt kann beispielsweise die spontane Atemaktivität eines mit der Beatmungsvorrichtung beatmeten Patienten sein.

Eine mögliche Art der effektiven Filterung ist die gleitende Mittelwertbildung. Da der Sprungfehler schnell, d. h. innerhalb von wenigen Sekunden, auftritt, reicht es aus, den gleitenden Mittelwert über einige wenige Änderungswerte oder/und Messsignalwert zu bilden, etwa über nicht mehr als 25, vorzugsweise nicht mehr als 20, beispielsweise über 16 Werte hinweg. Bevorzugt kann ein gleitender gewichteter Mittelwert gebildet werden, um etwa zu unterschiedlichen Zeiten stattfindende Atemhübe unterschiedlich zu gewichten. So kann es beispielsweise sinnvoll sein, bei der Berücksichtigung des gleitenden Mittelwerts die zeitlich unmittelbar der aktuellen Messung vorausgehenden Werte weniger stark zu berücksichtigen als zeitlich weiter zurückliegende Werte. Ebenso kann es sich auch positiv auf die Genauigkeit der Fehlermittlung auswirken, die zu filternden Werte zum Ende des für die Filterung zu betrachtenden Zeitfensters wiederum weniger zu gewichten als etwa in der Mitte des für die Filterung zu betrachtenden Zeitfensters gelegene Werte.

Auch für die Bestimmung des Driftfehlers können die Messsignalwerte der beiden Durchflusssensoren jeweils durch einen gleitenden Mittelwert, insbesondere durch einen gewichteten gleitenden Mittelwert, geglättet werden.

Zusätzlich oder alternativ können die zu glättenden Messsignalwerte auch durch einen digitalen Filter mit einer höheren Ordnung als 1 gefiltert werden, wobei durch eine Filterung mit einer Ordnung höher als 2, besonders bevorzugt mit einem digitalen Filter vierter Ordnung noch bessere Glättungseffekte ohne Genauigkeitsverlust bei der Fehlererfassung erzielt werden. Ein derartiger digitaler Filter kommt vorzugsweise bei der Ermittlung des Driftfehlers zum Einsatz.

Da der Driftfehler schleichend über einen längeren Zeitraum von mehreren Minuten auftritt, werden bei der Glättung der Messsignale durch einen, gegebenenfalls gewichteten, gleitenden Mittelwert oder durch den digitalen Filter bevorzugt eine größere Anzahl an Messsignalen einbezogen als bei der Glättung für die Ermittlung des Sprungfehlers. Vorzugsweise werden für die Glättung zur Ermittlung des Driftfehlers wenigstens die letzten 50 Messwerte, besonders bevorzugt wenigstens die letzten 75 Messwerte, höchstbevorzugt wenigstens die letzten 100 Messwerte herangezogen.

Die Steuereinrichtung ist dann bevorzugt dazu ausgebildet ist, die Änderungswerte auf Grundlage der geglätteten Messsignale zu ermitteln.

Es sei an dieser Stelle klargestellt, dass unter einem digitalen Filter erster Ordnung eine rekursive Filtervorschrift unter Verwendung eines Filterkoeffizienten a gemäß folgender Struktur verstanden wird:

$$y(n) = a \cdot y(n-1) + (1-a) \cdot x(n)$$

wobei $y(n)$ die Filterausgabe für den n-ten Atemhub ist, $y(n-1)$ die Filterausgabe für den unmittelbar vorhergehenden (n-1)-ten Atemhub ist und $x(n)$ das aktuelle Eingangssignal für den n-ten Atemhub ist. a ist ein für den Filter bestimmter Filterkoeffizient.

Ein Filter m-ter Ordnung ist dabei die m-fache Anwendung der oben bezeichneten Filterstruktur mit der Filterausgabe der i-ten Ordnung für den n-ten Atemhub als Eingangssignal des Filters der nächsthöheren (i+1)-ten Ordnung für den n-ten Atemhub, wobei $1 \leq i \leq m$ gilt.

Bevorzugt arbeiten der proximale und der distale Durchflusssensor nicht kontinuierlich, sondern zeitdiskret, sodass der proximale und der distale Durchflusssensor jeweils pro Atemhub einen, vorzugsweise genau einen, Messwert liefern, welcher den diesem Atemhub zugeordneten Gasfluss repräsentiert.

Um nicht nur einen Fehler zu erfassen, sondern im Falle einer Fehlererfassung hieraus auch Maßnahmen ableiten zu können, ist die Steuereinrichtung vorzugsweise dazu ausgebildet, dann, wenn sie auf einen Fehler schließt, einen Alarm auszugeben. Anders als im Stand der Technik ist es jedoch nicht notwendig, einen Sensor abzuschalten. Daher ist die Steuereinrichtung bevorzugt dazu ausgebildet, die Verarbeitung von Messsignalen des proximalen Durchflusssensors fortzusetzen und insbesondere weiterhin auf das Vorliegen eines Sensorfehlers zu überprüfen.

Die vorliegende Erfindung wird nachfolgend anhand der beiliegenden Zeichnungen näher erläutert werden. Es stellt dar:

Figur 1 eine erfindungsgemäße Ausführungsform einer Beatmungsvorrichtung der vorliegenden Erfindung,

Figur 2 ein Diagramm mit Rohmesssignalen und geglätteten Messsignalen des distalen und des proximalen Durchflusssensors,

Figur 3 ein Diagramm mit Änderungswerten der Messsignale des distalen und des proximalen Durchflusssensors,

Figur 4 ein weiteres Diagramm mit Rohmesssignalen und geglätteten Messsignalen des distalen und des proximalen Durchflusssensors,

Figur 5 ein weiteres Diagramm mit Änderungswerten der Messsignale des distalen und des proximalen Durchflusssensors,

Figur 6 ein grobschematisches Diagramm zur Fehlererfassung eines Sprungfehlers,

Figur 7 grobschematische Diagramme zur Fehlererfassung eines Driftfehlers.

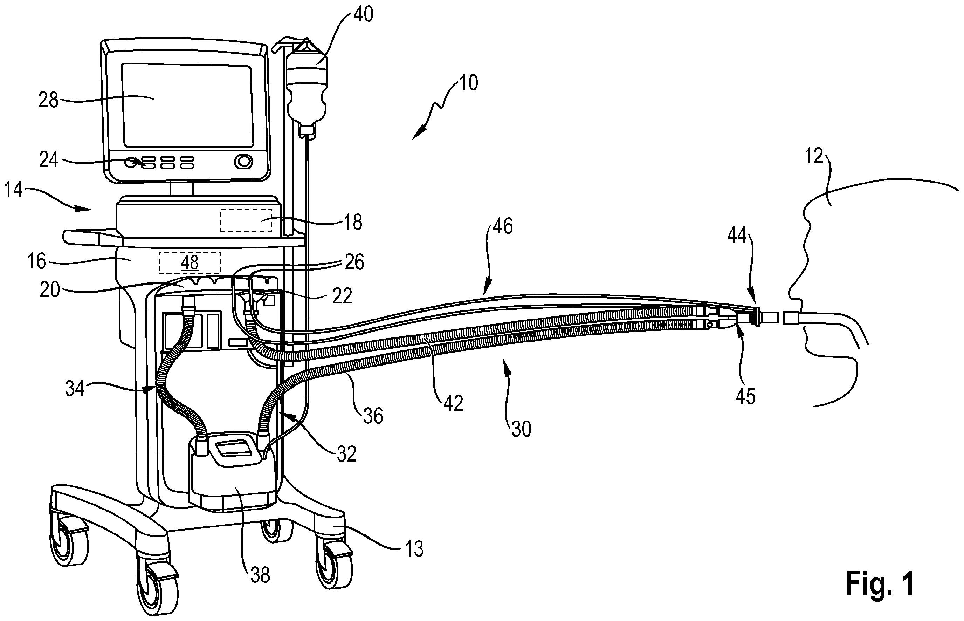

In Figur 1 ist eine erfindungsgemäße Ausführungsform einer Beatmungsvorrichtung allgemein mit 10 bezeichnet. Die Beatmungsvorrichtung 10 dient im dargestellten Beispiel zur künstlichen Beatmung eines humanen Patienten 12.

Lediglich der Vollständigkeit halber sei darauf hingewiesen, dass die erfindungsgemäße Beatmungsvorrichtung 10 als mobile Beatmungsvorrichtung 10 auf einem rollbaren Gestell 13 aufgenommen sein kann.

Die Beatmungsvorrichtung 10 weist ein Gehäuse 14 auf, in dem - von außen wegen des blickdichten Gehäusematerials nicht erkennbar - eine Druckveränderungsanordnung 16 und eine Steuereinrichtung 18 aufgenommen sein können.

Die Druckveränderungsanordnung 16 ist in an sich bekannter Weise aufgebaut und kann eine Pumpe, einen Verdichter, ein Gebläse, einen Druckbehälter, ein Reduzierventil und dergleichen aufweisen. Weiter weist die Beatmungsvorrichtung 10 in an sich bekannter Weise ein Inspirationsventil 20 und ein Exspirationsventil 22 auf.

Die Steuereinrichtung 18 ist üblicherweise als Computer oder Mikroprozessor realisiert. Sie umfasst eine in Figur 1 nicht dargestellte Speichereinrichtung, um für den Betrieb der Beatmungsvorrichtung 10 notwendige Daten speichern und erforderlichenfalls aufrufen zu können. Die Speichereinrichtung kann bei Netzwerkbetrieb auch außerhalb des Gehäuses 14 gelegen und durch eine Datenübertragungsverbindung mit der Steuereinrichtung 18 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist die Speichereinrichtung bevorzugt in die Steuereinrichtung 18 integriert oder wenigstens im selben Gehäuse 14 wie diese aufgenommen.

Zur Eingabe von Daten in die Beatmungsvorrichtung 10 bzw. genauer in die Steuereinrichtung 18 weist die Beatmungsvorrichtung 10 einen Dateneingang 24 auf, welcher in dem in Figur 1 dargestellten Beispiel durch eine Tastatur repräsentiert ist. Die Steuereinrichtung 18 kann alternativ oder zusätzlich zur dargestellten Tastatur Daten über verschiedene Dateneingänge erhalten, etwa über eine Netzwerkleitung, eine Funkstrecke oder über Sensoranschlüsse 26, auf die weiter unten im Einzelnen eingegangen wird.

Zur Ausgabe von Daten an den behandelnden Therapeuten kann die Beatmungsvorrichtung 10 ein Ausgabegerät 28 aufweisen, im dargestellten Beispiel einen Bildschirm.

Zur künstlichen Beatmung ist der Patient 12 mit der Beatmungsvorrichtung 10, genauer mit der Druckveränderungsanordnung 16 im Gehäuse 14, über eine Beatmungsleitungsanordnung 30 verbunden. Der Patient 12 ist hierfür intubiert.

Die Beatmungsleitungsanordnung 30 weist einen Inspirationsschlauch 32 auf, über welchen frisches Atemgas von der Druckveränderungsanordnung 16 in die Lunge des Patienten 12 geleitet werden kann. Der Inspirationsschlauch 32 kann unterbrochen sein und einen ersten Inspirationsteilschlauch 34 und einen zweiten Inspirationsteilschlauch 36 aufweisen, zwischen welchen eine Konditionierungseinrichtung 38 zur gezielten Befeuchtung und gegebenenfalls auch Temperierung des dem Patienten 12 zugeführten frischen Atemgases vorgesehen sein kann. Die Konditionierungseinrichtung 38 kann mit einem externen Flüssigkeitsvorrat 40 verbunden sein, über den Wasser zur Befeuchtung oder auch ein Medikament, etwa zur Entzündungshemmung oder zur Erweiterung der Atemwege, dem Atemgas zugeführt werden kann. Bei einem Einsatz der vorliegenden Beatmungsvorrichtung 10 als Anästhesie-Beatmungsvorrichtung können so volatile Anästhetika kontrolliert über die Beatmungsvorrichtung 10 an den Patienten 12 abgegeben werden. Die Konditionierungseinrichtung 38 sorgt dafür, dass das frische Atemgas dem Patienten 12 mit einem vorbestimmten Feuchtegehalt, gegebenenfalls unter Zugabe eines Medikamenten-Aerosols und mit einer vorbestimmten Temperatur zugeleitet wird.

Die Beatmungsleitungsanordnung 30 weist neben dem bereits erwähnten Inspirationsventil 20 ein Exspirationsventil 22 und weiter einen Exspirationsschlauch 42 auf, über welchen verstoffwechseltes Atemgas aus der Lunge des Patienten 12 in die Atmosphäre abgeblasen wird.

Der Inspirationsschlauch 32 ist mit dem Inspirationsventil 20 gekoppelt, der Exspirationsschlauch 42 mit dem Exspirationsventil 22. Von den beiden Ventilen ist jeweils nur eines gleichzeitig zum Durchlass einer Gasströmung geöffnet. Die Betätigungssteuerung der Ventile 20 und 22 erfolgt ebenfalls durch die Steuereinrichtung 18.

Während eines Beatmungszyklus ist zunächst für die Dauer der Inspirationsphase das Exspirationsventil 22 geschlossen und das Inspirationsventil 20 geöffnet, sodass frisches Atemgas vom Gehäuse 14 zum Patienten 12 geleitet werden kann. Eine Strömung des frischen Atemgases wird durch gezielte Druckerhöhung des Atemgases durch die Druckveränderungsanordnung 16 bewirkt. Aufgrund der Druckerhöhung strömt das frische Atemgas in die Lunge des Patienten 12 und expandiert dort den lungennahen Körperbereich, also insbesondere den Brustkorb, gegen die individuelle Elastizität der lungennahen Körperteile. Hierdurch steigt auch der Gasdruck im Inneren der Lunge des Patienten 12 an.

Am Ende der Inspirationsphase wird das Inspirationsventil 20 geschlossen und das Exspirationsventil 22 geöffnet. Es beginnt die Exspirationsphase. Aufgrund des bis zum Ende der Inspirationsphase erhöhten Gasdrucks des in der Lunge des Patienten 12 befindlichen Atemgases strömt dieses nach dem Öffnen des Exspirationsventils 22 in die Atmosphäre, wobei sich mit fortschreitender Strömungsdauer der Gasdruck in der Lunge des Patienten 12 verringert. Erreicht der Gasdruck in der Lunge 12 einen an der Beatmungsvorrichtung 10 eingestellten positiven end-exspiratorischen Druck, also einen geringfügig höheren Druck als den Atmosphärendruck, wird die Exspirationsphase mit dem Schließen des Exspirationsventils 22 beendet und es schließt sich ein weiterer Beatmungszyklus an.

Während der Inspirationsphase wird dem Patienten 12 das sogenannte Beatmungs-Tidalvolumen zugeführt, also das Atemgas-Volumen pro Atemhub. Das Beatmungs-Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuereinrichtung 18, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Hierzu kann die Beatmungsvorrichtung 10 mit einem oder mehreren Sensoren datenübertragungsmäßig verbunden sein, welche den Zustand des Patienten oder/und den Betrieb der Beatmungsvorrichtung überwachen.

Einer dieser Sensoren ist ein proximaler Durchflusssensor 44, welcher in einem Y-Verbindungsstück 45 die dort in der Beatmungsleitungsanordnung 30 herrschende Atemgas-Strömung erfasst. Der Durchflusssensor 44 kann mittels einer Sensor-Leitungsanordnung 46 mit den Dateneingängen 26 der Steuereinrichtung 18 gekoppelt sein. Die Sensor-Leitungsanordnung 46 kann, muss jedoch nicht, elektrische Signalübertragungsleitungen umfassen. Sie kann ebenso Schlauchleitungen aufweisen, die den in Strömungsrichtung beiderseits des Durchflusssensors 44 herrschenden Gasdruck an die Dateneingänge 26 übertragen, wo diese von in Figur 1 nicht dargestellten Drucksensoren quantifiziert werden. Der Durchflusssensor 44 ist vorliegend als Differenzdruck-Durchflusssensor 44 dargestellt. Der Durchflusssensor 44 ist zwar bevorzugt ein nach dem Differenzdruck-Prinzip arbeitender Durchflusssensor, kann jedoch auch ein nach einem anderen physikalischen Wirkprinzip arbeitender Durchflusssensor sein.

Im Gehäuse 14 ist ein weiterer Durchflusssensor 48 vorgesehen, welcher aufgrund seiner größeren Entfernung vom Patienten 12 - verglichen mit dem proximalen Durchflusssensor 44 - als distaler Durchflusssensor 48 bezeichnet ist.

Der distale Durchflusssensor 48 und sein Messsignal können beispielsweise zur Regelung des Atemgasflusses durch die Beatmungsleitungsanordnung 30, im Falle des distalen Durchflusssensors 48 genauer durch den Inspirationsschlauch 32, während einer Inspirationsphase dienen, während der proximale Durchflusssensor 44 und sein Messsignal zur Regelung des dem Patienten 12 zugeführten Minutenvolumens dienen können. Der distale Durchflusssensor 48 weist somit bevorzugt ein schnelleres Ansprechverhalten auf als der proximale Durchflusssensor 44, da das Messsignal des distalen Durchflusssensors 48 auch zur Veränderung des Atemgasflusses während eines Atemhubes dient, während das Messsignal des proximalen Durchflusssensors 44 bevorzugt nur atemhubsweise berücksichtigt wird, somit auf dessen Grundlage keine Veränderung des Atemgasflusses durch das Y-Verbindungsstück 45 während eines Atemhubs erfolgt.

Aufgrund des Ortes seiner Anbringung in dem Y-Verbindungsstück 45 ist der proximale Durchflusssensor 44 im Gegensatz zum distalen Durchflusssensor 48 grundsätzlich auch in der Lage, den Fluss von exspiratorischem Atemgas durch den Exspirationsschlauch 42 zu erfassen.

Die korrekte Funktion der Durchflusssensoren 44 und 48 ist für den korrekten Betrieb der Beatmungsvorrichtung 10 und somit für die Gesundheit des Patienten 12 wesentlich.

Es hat sich im Betrieb gezeigt, dass gerade der proximale Durchflusssensor 48 aufgrund seiner Nähe zum Patienten 12 einem größeren Fehlerrisiko unterliegt als der distale Durchflusssensor 48. Der proximale Durchflusssensor 44, durch den auch exspiratorisches Atemgas strömt, ist beispielsweise stärker als der distale Durchflusssensor 48 durch im Atemgas enthaltene Feuchtigkeit belastet. Dies gilt umso mehr, wenn der distale Durchflusssensor 48 wie im vorliegenden Beispiel der Figur 1 in Inspirationsrichtung stromaufwärts der Konditionierungseinrichtung 38 angeordnet ist und somit im Wesentlichen nur von trockenem inspiratorischem Atemgas durchströmt wird.

Die Steuereinrichtung 18 der erfindungsgemäßen Beatmungsvorrichtung 10 ist zur Überwachung des Betriebs der aus dem proximalen Durchflusssensor 44 und dem distalen Durchflusssensor 48 gebildeten Durchflusssensoranordnung ausgebildet, um eine Fehlfunktion der Durchflusssensoranordnung rechtzeitig erkennen zu können.

Gemäß der bisher gemachten Erfahrungen treten am proximalen Durchflusssensor 44 zwei sich hinsichtlich der Geschwindigkeit ihres Auftretens unterscheidende Fehlertypen auf, nämlich ein schnell innerhalb weniger Sekunden auftretender Sprungfehler und ein schleichend innerhalb mehrerer Minuten auftretender Driftfehler.

Zunächst soll die Erfassung des Sprungfehlers beschrieben werden.

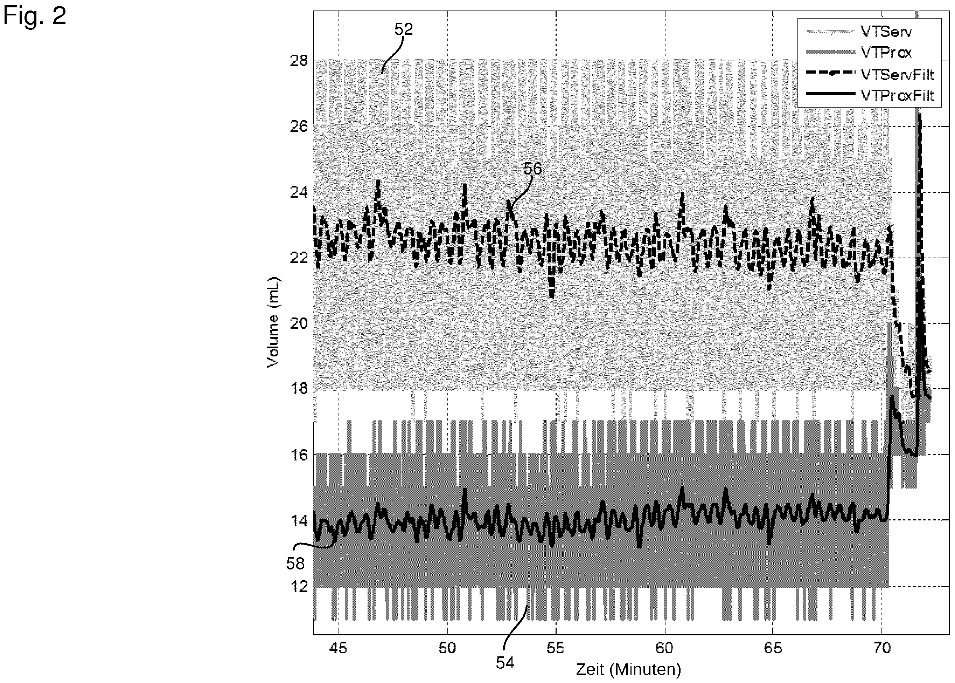

In Figur 2 sind die Erfassungssignale des proximalen Durchflusssensors 44 als "VTProx" mit Bezugszeichen 52 und des distalen Durchflusssensors 48 als "VTServ" mit Bezugszeichen 54 dargestellt. Es handelt sich dabei um zeitdiskrete Signale in ihrer unmittelbar von den jeweiligen Sensoren 44 und 48 erfassten Form.

Grundsätzlich können diese Signale zur Durchführung der erfindungsgemäßen Fehlererfassung verwendet werden. Zur Erhöhung der Erkennungssicherheit von Fehlern insbesondere am proximalen Durchflusssensor 44 empfiehlt es sich jedoch, diese Signale zu glätten.

Für die Erfassung des spontan auftretenden Sprungfehlers ist ein Glättverfahren vorteilhaft, welches nur einige wenige Messsignale berücksichtigt und somit kurzfristig auftretende Änderungen in den Sensorsignalen nicht unterdrückt. Als vorteilhaft hierfür hat sich die Glättung durch Bildung eines gewichteten gleitenden Mittelwerts erwiesen, etwa über die letzten 16 Messwerte hinweg. Die Messsignale des proximalen Durchflusssensors 44 und des distalen Durchflusssensors 48 werden dabei vorteilhaft mit den gleichen Messverfahren und den gleichen Filterkoeffizienten geglättet. Dies muss jedoch nicht so sein. Für die Messsignale unterschiedlicher Sensoren können unterschiedliche Glättungsverfahren oder das gleiche Glättungsverfahren mit unterschiedlichen Filterkoeffizienten verwendet werden. Im vorliegenden Fall eines gewichteten gleitenden Mittelwerts sind die Filterkoeffizienten die Gewichtungsfaktoren der jeweiligen Messwerte. Dies führt zu ungefilterten Messwerten, welche für den n-ten Atemhub des distalen Durchflusssensors 48 wie folgt aussehen können:

$$VT_{Serv_{Filt}}[n] = \frac{1}{64} \sum_{i=1}^{16} a_i \cdot VT_{Serv_{Filt}}[n-1]$$

mit $a_i$ = {1, 2, 3, 4, 5, 6, 7, 8, 8, 7, 6, 5, 4, 3, 2, 1}

Es werden bei dem angegebenen Beispiel die in der Mitte der 16 letzten Messwerte gelegenen Messwerte am stärksten gewichtet, mit einem linearen Anstieg vom jüngsten Messwert zu den mittleren Messwerten hin und mit einem linearen Abfall von den mittleren Messwerten zum ältesten Messwert. Entsprechend kann das zur Ermittlung eines Sprungfehlers geglättete Signal des proximalen Sensors wie folgt aussehen:

$$VT_{Prox_{Filt}}[n] = \frac{1}{64} \sum_{i=1}^{16} a_i \cdot VT_{Prox_{Filt}}[n-1]$$

wobei die Gewichtungsfaktoren dieselben sind.

Der gewichtete geglättete Mittelwert $VT_{SerfFilt}$ ist in Figur 2 mit dem Bezugszeichen 56, der gewichtete geglättete Mittelwert $VT_{ProxFilt}$ mit dem Bezugszeichen 58 versehen.

Die Abszisse des Diagramms von Figur 2 bezeichnet die Zeit in Minuten, die Ordinate das Volumen in Millilitern.

Die für die Fehlererfassung der erfindungsgemäßen Beatmungsvorrichtung wichtigen Änderungswerte werden besonders vorteilhaft als Differenzen des Werts des aktuellen Atemhubs und des unmittelbar vorhergehenden Atemhubs verwendet, wobei bevorzugt die geglätteten Werte zur Bildung der Differenzen herangezogen werden. Die Änderungs-

werte VT$_{\text{SerfDiff}}$ des distalen Durchflusssensors 48 und VT$_{\text{ProxDiff}}$ des proximalen Durchflusssensors 44 können daher wie folgt gebildet werden:

$$VT_{Serv_{Diff}}[n] = VT_{Serv_{Filt}}[n] - VT_{Serv_{Filt}}[n-1]$$

$$VT_{Prox_{Diff}}[n] = VT_{Prox_{Filt}}[n] - VT_{Prox_{Filt}}[n-1]$$

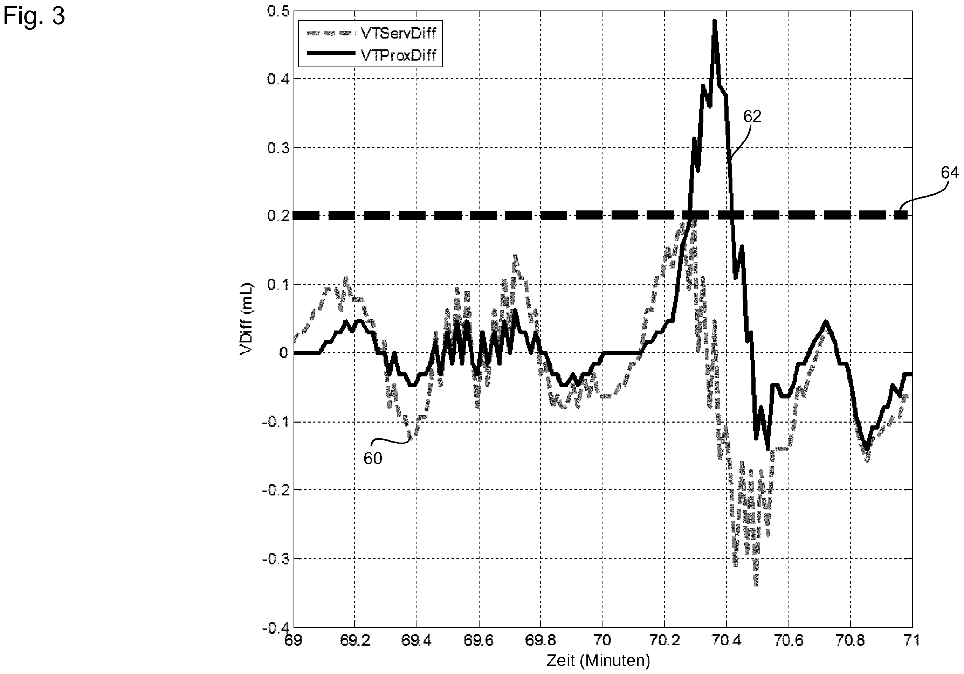

Diese Änderungswerte sind in ihrem zeitlichen Verlauf in Figur 3 gezeigt. Der Änderungswert des distalen Durchflusssensors 48 ist dabei mit dem Bezugszeichen 60, jener des proximalen Durchflusssensors 44 mit dem Bezugszeichen 62 gekennzeichnet. Wiederum zeigt die Abszisse des Diagramms von Figur 3 die Zeit in Minuten und die Ordinate ein Volumen, diesmal das Änderungsvolumen, in Millilitern.

In Figur 3 ist mit Bezugszeichen 64 ein Schwellenwert bezeichnet, bei dessen Überschreitung durch den Änderungswert 62 des proximalen Durchflusssensors 44 die Steuereinrichtung 18 mit dem in der Beschreibungseinleitung geschilderten Fehlererfassungsverfahren beginnt. Der Start des Fehlererfassungsverfahrens ist an das Überschreiten des Schwellenwerts 64 gekoppelt, um nicht unnötig Rechenleistung der Steuereinrichtung 18 für das Fehlererfassungsverfahren aufzuwenden.

Wenn beim k-ten Atemhub der Änderungswert 62 des proximalen Durchflusssensors 44 der Schwellenwert 64 vom Änderungswert 62 des proximalen Durchflusssensors 44 überschritten wird, werden die Änderungswerte der beiden Durchflusssensoren 44 und 48 des unmittelbar vorhergehenden (k-1)-ten Atemhubs jeweils als Bezugsgrößen für einen späteren Vergleich gespeichert:

$$VT_{Serv_{Bezug}} = VT_{Serv_{Filt}}[k-1]$$

$$VT_{Prox_{Bezug}} = VT_{Prox_{Filt}}[k-1]$$

Es wird dann mit einer Aufsummierung der Änderungswerte begonnen:

$$VT_{Serv_{Summe}}[n] = VT_{Serv_{Summe}}[n-1] + VT_{Serv_{Diff}}[n]$$

$$VT_{Prox_{Summe}}[n] = VT_{Prox_{Summe}}[n-1] + VT_{Prox_{Diff}}[n]$$

Für jeden Durchflusssensor 44 und 48 wird somit ein Summenwert gebildet, der für den n-ten Atemhub der Summenwert des unmittelbar vorhergehenden (n-1)-ten Atemhubs zuzüglich des aktuellen Änderungswerts des n-ten Atemhubs ist.

Auf einen Sprungfehler erkennt die Steuereinrichtung 18 im dargestellten Ausführungsbeispiel dann, wenn das Verhältnis des Summenwerts des proximalen Durchflusssensors 44 zum Bezugswert VT$_{ProxBezug}$ des proximalen Durchflusssensors 44 einen vorbestimmten Bezugsschwellenwert überschreitet, beispielsweise einen Bezugsschwellenwert von 0,08 überschreitet, was bedeutet, dass die Summe der Änderungswerte des proximalen Durchflusssensors seit Beginn der Aufsummierung wenigstens 8 % des Bezugswerts beträgt. Der Bezugsschwellenwert kann auch einen anderen Wert als 8 % aufweisen, jedoch haben sich in bisherigen Versuchen 8 % als sehr guter Bezugsschwellenwert erwiesen.

Als weitere Bedingung, welche zu der im vorhergehenden Absatz genannten hinzutreten kann, ist, dass sich der Summenwert des distalen Durchflusssensors 48 um nicht mehr als einen weiteren Bezugsschwellenwert - dieser kann beispielsweise bei 10 % liegen - vom Summenwert des proximalen Durchflusssensors 44 unterscheidet.

Alternativ oder zusätzlich zu der im vorhergehenden Absatz genannten weiteren Bedingung kann die Bedingung hinzutreten, dass sich der aktuelle, vorzugsweise geglättete, Wert des distalen Durchflusssensors 48 nicht mehr als um einen noch weiteren Bezugsschwellenwert von seinem Bezugswert unterscheidet. Dieser noch weitere Bezugsschwellenwert kann beispielsweise zwischen 1 und 5 % liegen. Versuche haben einen noch weiteren Bezugsschwellenwert von 2 % als zielführend herausgestellt.

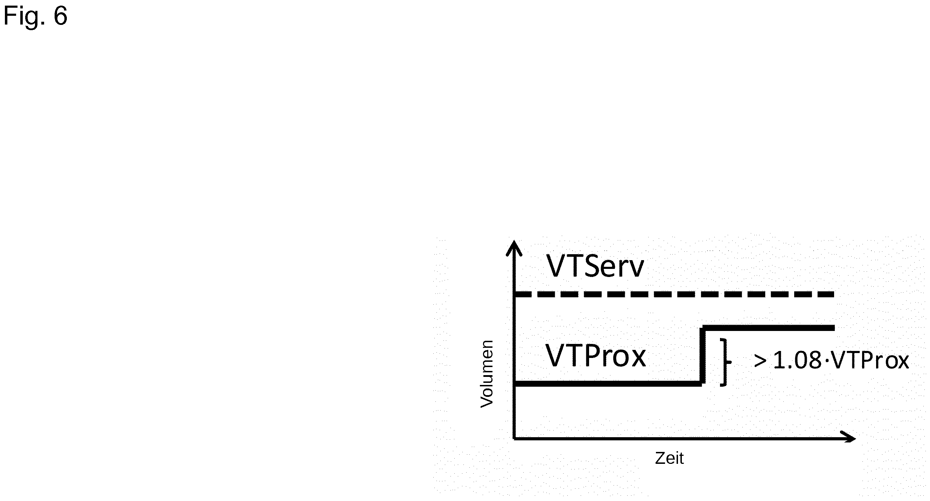

Der Fall eines am proximalen Durchflusssensor 44 auftretenden Sprungfehlers ist in Figur 6 beispielhaft thematisch gezeigt.

Erst wenn alle für eine zuverlässige Erkennung eines Sprungfehlers vorbestimmten Bedingungen erfüllt sind, erkennt die Steuereinrichtung 18 auf Vorhandensein eines Sprungfehlers und löst beispielsweise einen entsprechenden Alarm aus.

Für die Erkennung eines schleichend auftretenden Driftfehlers werden ebenfalls geglättete Messwerte verwendet, wobei vorzugsweise die Glättung der Messwerte über eine größere Anzahl an zurückliegenden Messwerten erfolgt, als dies beim Sprungfehler der Fall ist. Beispielsweise kann die Glättung wieder durch Bildung eines gewichteten gleitenden Mittelwerts erfolgen, etwa über die 100 letzten Messwerte hinweg:

$$VT_{Serv_{Filt}}[n] = \sum_{i=1}^{100} b_i \cdot VT_{Serv_{Filt}}[n-1]$$

$$VT_{Prox_{Filt}}[n] = \sum_{i=1}^{100} b_i \cdot VT_{Prox_{Filt}}[n-1]$$

Die Gewichtungskoeffizienten $b_i$ sind dabei so gewählt, dass die Summe aller Gewichtungskoeffizienten 1 ergibt. Alternativ oder zusätzlich können die Messwerte auch durch einen digitalen Filter höherer Ordnung, vorzugsweise wenigstens vierter Ordnung, geglättet werden.

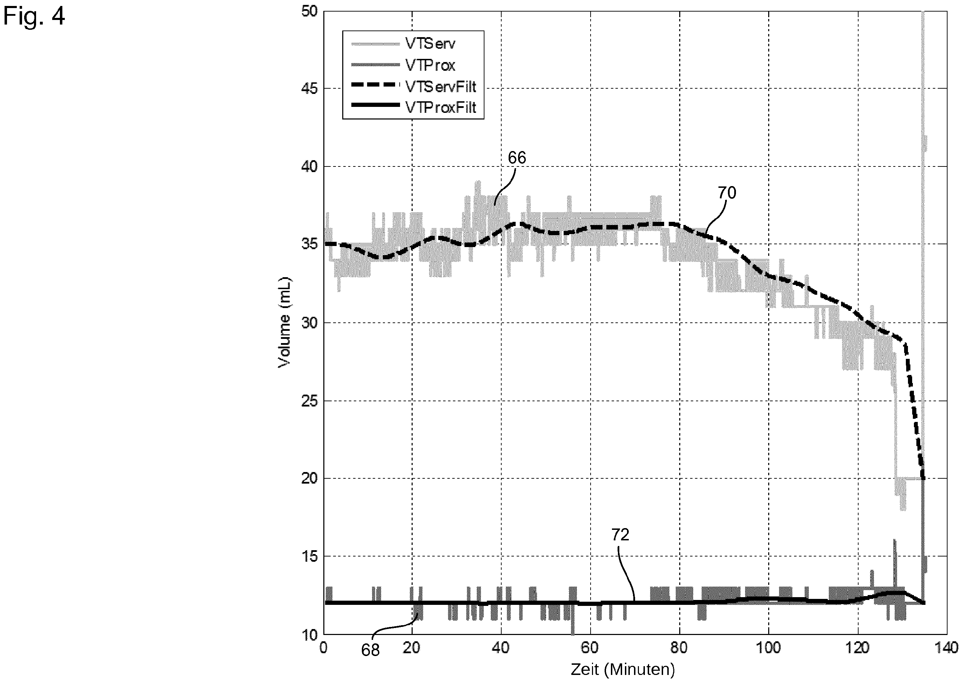

In Figur 4 sind die Rohsignale des distalen Durchflusssensors 48 mit Bezugszeichen 66 versehen und jene des proximalen Durchflusssensors 44 mit Bezugszeichen 68. Das geglättete Signal des distalen Durchflusssensors 48 ist dagegen mit Bezugszeichen 70 versehen, das geglättete Signal des proximalen Durchflusssensors 44 mit Bezugszeichen 72.

Die Bildung der Änderungswerte der Messsignale 66 und 68 des distalen bzw. proximalen Durchflusssensors 48 bzw. 44 erfolgt wie oben bereits beschrieben, nämlich durch Bildung der Differenz zwischen dem geglätteten Messwert des jeweiligen Sensors für den aktuellen Atemhub und jenem des unmittelbar vorhergehenden Atemhubs. Es gilt also wiederum:

$$VT_{Serv_{Diff}}[n] = VT_{Serv_{Filt}}[n] - VT_{Serv_{Filt}}[n-1]$$

$$VT_{Prox_{Diff}}[n] = VT_{Prox_{Filt}}[n] - VT_{Prox_{Filt}}[n-1]$$

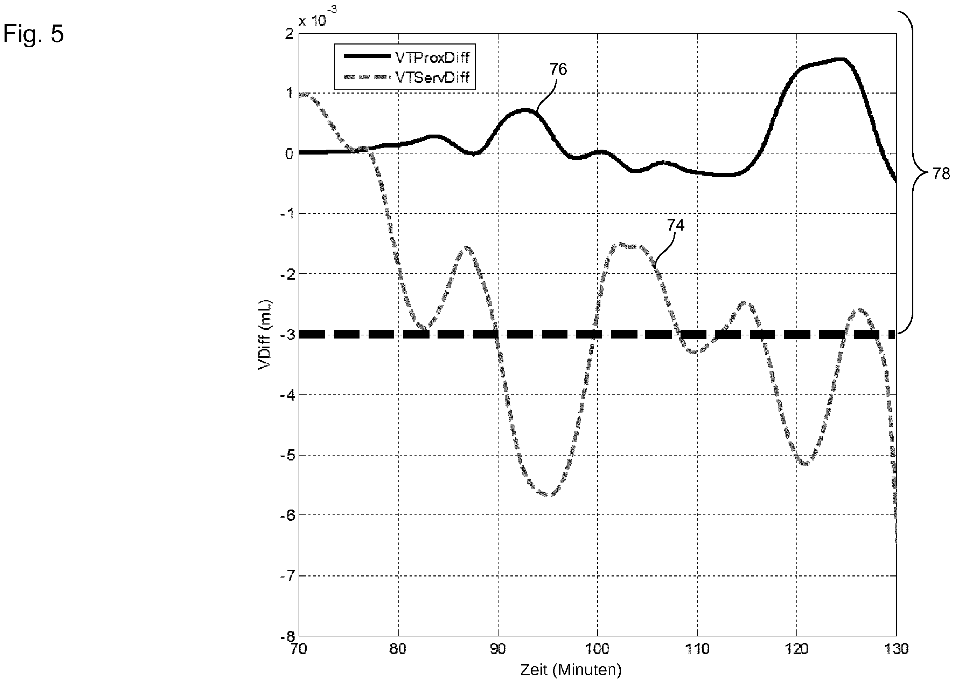

Die Änderungswerte der Messsignale des distalen Durchflusssensors 48 sind in Figur 5 mit Bezugszeichen 74 bezeichnet, jene des proximalen Durchflusssensors 44 mit Bezugszeichen 76.

Wiederum beginnt die Fehlererfassung durch die Steuereinrichtung 18 erst, nachdem vorbestimmte Startbedingungen erfüllt sind, etwa dann, wenn einer der Änderungswerte ein in Figur 5 mit 78 bezeichnetes Toleranzband um 0 ml herum verlässt oder/und wenn die Änderungswerte unterschiedliche Vorzeichen aufweisen. In Figur 5 ist der obere Rand des Toleranzbandes nicht dargestellt, es reicht von -3 bis +3 ml. Das Änderungswerte-Toleranzband, dessen Verlassen eine Startbedingung für die Fehlererfassung durch die Steuereinrichtung 18 sein kann, ist vorzugsweise symmetrisch um den Nullpunkt herum angeordnet. Dies muss jedoch nicht so sein, es kann je nach Anwendungsfall auch betragsmäßig voneinander abweichende Grenzen haben.

Dann, wenn die vorbestimmte Startbedingung für die Erfassung eines Driftfehlers vorliegt, beginnt die Steuereinrichtung 18 wiederum, in der bereits oben beschriebenen Weise für die Änderungswerte eines jeden Durchflusssensors 44 bzw. 48 Summenwerte zu bilden:

$$VT_{Serv_{Summe}}[n] = VT_{Serv_{Summe}}[n-1] + VT_{Serv_{Diff}}[n]$$

$$VT_{Prox_{Summe}}[n] = VT_{Prox_{Summe}}[n-1] + VT_{Prox_{Diff}}[n]$$

Ebenso werden die Messwerte der beiden Sensoren 44 und 48 desjenigen Atemhubs, welcher jenem Atemhub unmittelbar vorausging, bei dem die wenigstens eine Startbedingung einer Fehlererfassung erfüllt wurde, als Bezugswerte für spätere Vergleiche gespeichert:

$$VT_{Serv_{Bezug}} = VT_{Serv_{Filt}}[k-1]$$

$$VT_{Prox_{Bezug}} = VT_{Prox_{Filt}}[k-1]$$

Hier unterscheidet sich die Fehlererfassung eines Driftfehlers nicht von der Fehlererfassung eines Sprungfehlers.

Die Steuereinrichtung 18 erkennt im vorliegenden Beispiel dann auf Vorliegen eines Driftfehlers in der Durchflusssensoranordnung, wenn das Verhältnis des Änderungssummenwerts $VT_{ProxSumme}[n]$ des proximalen Durchflusssensors 44 zu dessen Bezugswert $VT_{Prox_{Bezug}}$ einen vorbestimmten Bezugsschwellenwert übersteigt und wenn gleichzeitig das Verhältnis des Änderungssummenwerts $VT_{ServSumme}[n]$ des distalen Durchflusssensors 48 zu dessen Bezugswert $VT_{Serv_{Bezug}}$ einen vorbestimmten weiteren Bezugsschwellenwert nicht übersteigt. Der Bezugsschwellenwert des proximalen Durchflusssensors 44 für die Erfassung eines Driftfehlers kann wiederum bei 8 % liegen, der weitere Bezugsschwellenwert kann beispielsweise bei 2 % liegen.

Ebenso erkennt die Steuereinrichtung 18 im vorliegenden Beispiel dann auf Vorliegen eines Driftfehlers in der Durchflusssensoranordnung, wenn das Verhältnis des Änderungssummenwerts $VT_{ServSumme}[n]$ des distalen Durchflusssensors 48 zu dessen Bezugswert $VT_{Serv_{Bezug}}$ den vorbestimmten Bezugsschwellenwert übersteigt und wenn gleichzeitig das Verhältnis des Änderungssummenwerts des proximalen Durchflusssensors 44 $VT_{ProxSumme}[n]$ zu dessen Bezugswert $VT_{Prox_{Bezug}}$ den vorbestimmten weiteren Bezugsschwellenwert nicht übersteigt. Es gelten dabei die im vorhergehenden Absatz angegebenen beispielhaften Werte für die Bezugsschwellenwerte.

Da der Driftfehler sich am proximalen oder am distalen Durchflusssensor auswirken kann, sind die Bedingungen für die Erfassung des Driftfehlers bevorzugt symmetrisch bezüglich der beiden Bezugsschwellenwerte.

Als zusätzliche Bedingung für die Erfassung eines Driftfehlers kann hinzutreten, dass einer der beiden Änderungssummenwerte eine vorbestimmte Schwellenänderungsmenge überschreitet, beispielsweise eine Menge von 2 ml.

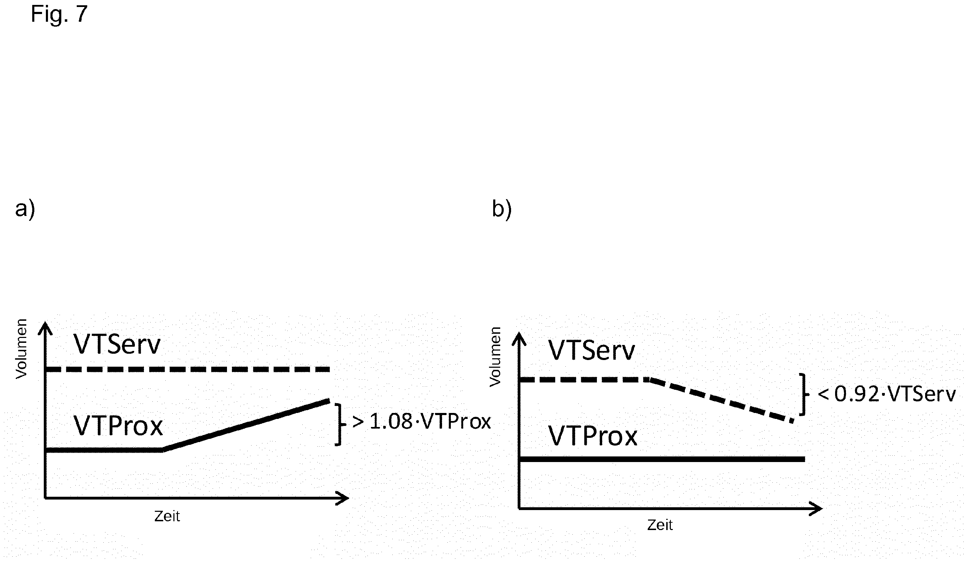

Die Bedingungen zur Erfassung eines Driftfehlers anhand der Änderungssummenwerte sind in Figur 7a und 7b grobschematisch dargestellt. Im Falle einer Erkennung eines Driftfehlers löst die Steuereinrichtung 18 vorzugsweise einen Alarm aus, betreibt die Durchflusssensoranordnung jedoch bevorzugt weiter.

Mit der vorliegenden erfindungsgemäßen Beatmungsvorrichtung 10 können Fehler in der Durchflusssensoranordnung umfangreicher und zielgenauer als bisher erfasst werden.

**Patentansprüche**

1. Beatmungsvorrichtung (10) zur künstlichen Beatmung mit

   - einer Beatmungsgasquelle (16),
   - einer zwischen der Beatmungsgasquelle (16) und einem patientenseitigen, proximalen Ende verlaufenden Beatmungsleitungsanordnung (30),
   - einer Ventilanordnung (20, 22), umfassend ein Inspirationsventil (20) und ein Exspirationsventil (22),
   - einer Durchflusssensoranordnung (44, 48) zur quantitativen Erfassung eines Gasflusses in der Beatmungsleitungsanordnung (30), umfassend einen weiter vom patientenseitigen Ende der Beatmungsleitungsanordnung (30) entfernt angeordneten distalen Durchflusssensor (48) und einen näher beim patientenseitigen Ende der Beatmungsleitungsanordnung (30) gelegenen proximalen Durchflusssensor (44), und mit
   - einer Steuereinrichtung (18) wenigstens zur Verarbeitung von Messsignalen der Durchflusssensoranordnung (44, 48), wobei die Steuereinrichtung (18) dazu ausgebildet ist, auf Grundlage von Messsignalen des distalen (48) oder/und des proximalen Sensors (44) auf einen Fehler zu schließen,

   wobei die Steuereinrichtung (18) dazu ausgebildet ist, auf einen Fehler der Durchflusssensoranordnung (44, 48) zu schließen auf Grundlage eines Vergleichs eines Änderungswerts (62, 76) eines Messsignals (54, 58, 68, 72) des einen Durchflusssensors (44) aus proximalem Durchflusssensor (44) und distalem Durchflusssensor (48) mit

   - einem Änderungswert (60, 74) eines Messsignals (52, 56, 66, 70) des jeweils anderen Durchflusssensors (48)

oder/und mit

- einem Messsignal (54, 58, 68, 72) des einen Durchflusssensors (44),

**dadurch gekennzeichnet, dass** die Steuereinrichtung (18) dazu ausgebildet ist, dann auf einen Fehler der Durchflusssensoranordnung (44, 48) zu schließen, wenn

- ein Verhältnis eines Summenwerts von nacheinander anfallenden Änderungswerten (62, 76) des Messsignals (54, 58, 68, 72) des proximalen Durchflusssensors (44) zu einer ersten Bezugsgröße einen vorbestimmten Bezugsschwellenwert übersteigt und
- wenn ein Verhältnis eines Summenwerts von nacheinander anfallenden Änderungswerten (60, 74) des Messsignals (52, 56, 66, 70) des distalen Durchflusssensors (48) zu einer zweiten Bezugsgröße einen vorbestimmten Bezugsschwellenwert unterschreitet.

2. Beatmungsvorrichtung nach Anspruch 1,
**dadurch gekennzeichnet, dass** die Steuereinrichtung (18) dazu ausgebildet ist, dann auf einen Fehler der Durchflusssensoranordnung (44, 48) zu schließen, wenn der Änderungswert (62, 76 oder 60, 74) des Messsignals (54, 58, 68, 72 oder 52, 56, 66, 70) des einen Durchflusssensors (44 oder 48) aus distalem (48) und proximalem Durchflusssensor (44) eine entgegengesetzte Änderungsrichtung aufweist als der Änderungswert (60, 74 oder 62, 76 60, 74) des jeweils anderen Durchflusssensors (48 oder 44).

3. Beatmungsvorrichtung nach einem der vorhergehenden Ansprüche,
**dadurch gekennzeichnet, dass** die Änderungswerte (62, 76) des Messsignals (54, 58, 68, 72) des proximalen Durchflusssensors (44) unmittelbar aufeinander folgen oder/und dass die Änderungswerte (60, 74) des Messsignals (52, 56, 66, 70) des distalen Durchflusssensors (48) unmittelbar aufeinander folgen.

4. Beatmungsvorrichtung nach einem der vorhergehenden Ansprüche,
**dadurch gekennzeichnet, dass** die erste oder/und die zweite Bezugsgröße ein Messsignalwert des proximalen Durchflusssensors (44) ist, insbesondere ein Messsignalwert des proximalen Durchflusssensors (44) bei oder unmittelbar vor dem Beginn der Aufsummierung der Änderungswerte (62, 76, 60, 74) ist.

5. Beatmungsvorrichtung nach einem der vorhergehenden Ansprüche,
**dadurch gekennzeichnet, dass** die Steuereinrichtung (18) dazu ausgebildet ist, mit der Aufsummierung von Änderungswerten (62, 76, 60, 74) zu beginnen, wenn der Messsignalwert oder/und der Änderungswert des proximalen Durchflusssensors (44) einen vorbestimmten Signalschwellenwert (64) übersteigt.

6. Beatmungsvorrichtung nach Anspruch 5,
**dadurch gekennzeichnet, dass** die Steuereinrichtung (18) dazu ausgebildet ist, die Messsignale des proximalen (44) und des distalen Durchflusssensors (48) atemhubsweise zu verarbeiten, wobei bevorzugt dann, wenn der Messsignalwert des proximalen Durchflusssensors (44) den vorbestimmten Signalschwellenwert (64) bei einem Schwellen-Atemhub übersteigt, die erste oder/und die zweite Bezugsgröße der Messsignalwert des proximalen Durchflusssensors (48) ist, welcher dem dem Schwellen-Atemhub unmittelbar vorhergehenden Atemhub zugeordnet ist.

7. Beatmungsvorrichtung nach einem der vorhergehenden Ansprüche,
**dadurch gekennzeichnet, dass** die Steuereinrichtung (18) dazu ausgebildet ist, dann auf einen Fehler der Durchflusssensoranordnung (44, 48) zu schließen, wenn

- ein Verhältnis eines Summenwerts von nacheinander anfallenden, vorzugsweise unmittelbar aufeinanderfolgenden, Änderungswerten des Messsignals des proximalen Durchflusssensors (44) zu einer dritten Bezugsgröße einen vorbestimmten Bezugsschwellenwert übersteigt und
- wenn ein Verhältnis eines Summenwerts von nacheinander anfallenden, vorzugsweise unmittelbar aufeinanderfolgenden, Änderungswerten des Messsignals des distalen Durchflusssensors (48) zu einer vierten Bezugsgröße einen vorbestimmten Bezugsschwellenwert unterschreitet,

oder dann auf einen Fehler der Durchflusssensoranordnung (44, 48) zu schließen, wenn

- ein Verhältnis eines Summenwerts von nacheinander anfallenden, vorzugsweise unmittelbar aufeinanderfolgenden, Änderungswerten des Messsignals des proximalen Durchflusssensors (44) zu einer fünften Bezugsgröße einen vorbestimmten Bezugsschwellenwert unterschreitet und

- wenn ein Verhältnis eines Summenwerts von nacheinander anfallenden, vorzugsweise unmittelbar aufeinanderfolgenden, Änderungswerten des Messsignals des distalen Durchflusssensors (48) zu einer sechsten Bezugsgröße einen vorbestimmten Bezugsschwellenwert übersteigt.

**8.** Beatmungsvorrichtung nach Anspruch 7,
**dadurch gekennzeichnet, dass** die Steuereinrichtung (18) dazu ausgebildet ist, nur dann auf einen Fehler der Durchflusssensoranordnung (44, 48) zu schließen, wenn zusätzlich

- der Summenwert von nacheinander anfallenden, vorzugsweise unmittelbar aufeinanderfolgenden, Änderungswerten des Messsignals des proximalen Durchflusssensors (44) einen vorbestimmten ersten Summenschwellenwert übersteigt,
oder/und
- der Summenwert von nacheinander anfallenden, vorzugsweise unmittelbar aufeinanderfolgenden, Änderungswerten des Messsignals des distalen Durchflusssensors (48) einen vorbestimmten zweiten Summenschwellenwert übersteigt.

**9.** Beatmungsvorrichtung nach Anspruch 7 oder 8,
**dadurch gekennzeichnet, dass** die dritte oder/und die fünfte Bezugsgröße ein Messsignalwert des proximalen Durchflusssensors (44) ist, insbesondere ein Messsignalwert des proximalen Durchflusssensors (44) bei oder unmittelbar vor dem Beginn der Aufsummierung der Änderungswerte ist.

**10.** Beatmungsvorrichtung nach einem der Ansprüche 7 bis 9,
**dadurch gekennzeichnet, dass** die vierte oder/und die sechste Bezugsgröße ein Messsignalwert des distalen Durchflusssensors (48) ist, insbesondere ein Messsignalwert des distalen Durchflusssensors (48) bei oder unmittelbar vor dem Beginn der Aufsummierung der Änderungswerte ist.

**11.** Beatmungsvorrichtung nach einem der Ansprüche 7 bis 10,
**dadurch gekennzeichnet, dass** die Aufsummierung von Änderungswerten zur Bildung von Summenwerten beginnt, wenn der Betrag des Änderungswerts des Messsignals des proximalen (44) oder/und des distalen Durchflusssensors (48) einen vorbestimmten Betragsschwellenwert (78) übersteigt.

**12.** Beatmungsvorrichtung nach einem der vorhergehenden Ansprüche,
**dadurch gekennzeichnet, dass** die Steuereinrichtung (18) dazu ausgebildet ist, die Messsignale der Durchflusssensoren (44, 48) durch Filterung zu glätten, insbesondere durch gleitende Mittelwertbildung, bevorzugt durch Bildung eines gleitenden gewichteten Mittelwerts, oder durch einen digitalen Filter mit einer höheren Ordnung als 1, vorzugsweise als 2, besonders bevorzugt mit einem digitalen Filter vierter Ordnung, wobei die Steuereinrichtung (18) bevorzugt weiter dazu ausgebildet ist, die Änderungswerte auf Grundlage der geglätteten Messsignale zu ermitteln.

**13.** Beatmungsvorrichtung nach einem der vorhergehenden Ansprüche,
**dadurch gekennzeichnet, dass** der proximale (44) und der distale Durchflusssensor (48) jeweils pro Atemhub einen, vorzugsweise genau einen, Messwert liefern, welcher den diesem Atemhub zugeordneten Gasfluss repräsentiert.

**14.** Beatmungsvorrichtung nach einem der vorhergehenden Ansprüche,
**dadurch gekennzeichnet, dass** der proximale Durchflusssensor (44) ein Differenzdrucksensor ist.

**15.** Beatmungsvorrichtung nach einem der vorhergehenden Ansprüche,
**dadurch gekennzeichnet, dass** die Steuereinrichtung (18) dazu ausgebildet ist, dann, wenn sie auf einen Fehler schließt, einen Alarm auszugeben und vorzugsweise die Verarbeitung von Messsignalen des proximalen Durchflusssensors (44) fortsetzt.

**Claims**

**1.** Ventilation device (10) for artificial respiration with

- a breathing gas source (16),
- a ventilation line arrangement (30) extending between the breathing gas source (16) and a patient-side proximal end,
- a valve assembly (20, 22) comprising an inspiratory valve (20) and an expiratory valve (22),
- a flow sensor arrangement (44, 48) for the quantitative detection of a gas flow in the ventilation line arrangement (30) comprising a distal flow sensor (48) located farther from the patient end of the ventilation line arrangement (30) and a proximal flow sensor (44) located closer to the patient end of the ventilation line arrangement (30), and with
- a control device (18) at least for processing measurement signals of the flow sensor arrangement (44, 48), wherein the control device (18) is adapted to error detection based on measurement signals of the distal (48) or/and the proximal sensor (44),

wherein the control device (18) is adapted to detect an error of the flow sensor arrangement (44, 48) based on a comparison of a change value (62, 76) of a measurement signal (54, 58, 68, 72) of one flow sensor (44) out of the distal flow sensor (48) and the proximal flow sensor (44) with

- a change value (60, 74) of a measurement signal (52, 56, 66, 70) of the respective other flow sensor (48) or/and with
- a measurement signal (54, 58, 68, 72) of the one flow sensor (44),

**characterized in that** the control device (18) is adapted to detect an error of the flow sensor arrangement (44, 48) when

- a ratio of a sum value of consecutively occurring change values (62, 76) of the measurement signal (54, 58, 68, 72) of the proximal flow sensor (44) to a first reference variable exceeds a predetermined reference threshold value, and
- a ratio of a sum value of consecutively occurring change values (60, 74) of the measurement signal (52, 56, 66, 70) of the distal flow sensor (48) to a second reference variable falls below a predetermined reference threshold value.

2. A ventilation device according to claim 1,
**characterized in that** the control device (18) is adapted to detect an error of the flow sensor arrangement (44, 48) when the change value (62, 76 or 60, 74) of the measurement signal (54, 58 , 68, 72 or 52, 56, 66, 70) of the one flow sensor (44 or 48) out of the distal (48) and proximal flow sensor (44) has an opposite direction of change than the change value (60, 74 or 62, 76 60, 74) of the respective other flow sensor (48 or 44).

3. Ventilator according to one of the preceding claims,
**characterized in that** the change values (62, 76) of the measurement signal (54, 58, 68, 72) of the proximal flow sensor (44) occur immediately successively or/and that the change values (60, 74) of the measurement signal (52, 56, 66, 70) of the distal flow sensor (48) occur immediately successively.

4. A ventilator according to claim 3,
**characterized in that** the first or/and the second reference variable is a measurement signal value of the proximal flow sensor (44), in particular a measurement signal value of the proximal flow sensor (44) at or immediately before the beginning of the summation of the change values (62, 76 , 60, 74).

5. Ventilator according to one of claims 3 or 4,
**characterized in that** the control device (18) is adapted to start adding change values (62, 76, 60, 74) when the measurement signal value or/and the change value of the proximal flow sensor (44) exceeds a predetermined signal threshold (64).

6. Ventilator according to claim 5,
**characterized in that** the control device (18) is adapted to process the measurement signals of the proximal (44) and the distal flow sensor (48) on by-breath basis, wherein preferably when the measurement signal value of the proximal flow sensor (44) exceeds the predetermined signal threshold (64) at a threshold breath, the first or/and second reference variable is the measurement signal value of the proximal flow sensor (48), which is associated with the breath immediately preceding the threshold breath.

**7.** Ventilator according to one of the preceding claims,
**characterized in that** the control device (18) is adapted to detect an error of the flow sensor arrangement (44, 48) when

- a ratio of a sum value of consecutively occurring, preferably immediately successive, change values of the measurement signal of the proximal flow sensor (44) to a third reference variable exceeds a predetermined reference threshold value, and
- a ratio of a sum value of successively occurring, preferably immediately successive, change values of the measurement signal of the distal flow sensor (48) to a fourth reference variable falls below a predetermined reference threshold value,

or to detect an error in the flow sensor arrangement (44, 48) when

- a ratio of a sum value of consecutively occurring, preferably immediately successive, change values of the measurement signal of the proximal flow sensor (44) to a fifth reference value falls below a predetermined reference threshold and
- a ratio of a sum value of consecutively occurring, preferably immediately successive, change values of the measurement signal of the distal flow sensor (48) to a sixth reference value exceeds a predetermined reference threshold value.

**8.** Ventilation device according to claim 7,
**characterized in that** the control device (18) is adapted to detect an error of the flow sensor arrangement (44, 48), only if in addition

- the sum value of consecutively occurring, preferably immediately successive, change values of the measurement signal of the proximal flow sensor (44, 48) exceeds a predetermined first sum threshold value, or/and
- the sum value of consecutively occurring, preferably immediately successive, change values of the measurement signal of the distal flow sensor (48) exceeds a predetermined second sum threshold value.

**9.** Ventilator according to claim 7 or 8,
**characterized in that** the third or/and the fifth reference variable is a measurement signal value of the proximal flow sensor (44), in particular a measurement signal value of the proximal flow sensor (44) at or immediately before the beginning of the summation of the change values.

**10.** Ventilator according to one of claims 7 to 9,
**characterized in that** the fourth or/and the sixth reference value is a measurement signal value of the distal flow sensor (48), in particular a measurement signal value of the distal flow sensor (48) at or immediately before the beginning of the summation of the change values.

**11.** Ventilator according to one of claims 7 to 10,
**characterized in that** the summation of change values to form summation values begins when the absolute value of the change value of the measurement signal of the proximal (44) or/and the distal flow sensor (48) exceeds a predetermined absolute value threshold value (78).

**12.** Ventilator according to one of the preceding claims,
**characterized in that** the control device (18) is adapted to smooth the measurement signals of the flow sensors (44, 48) by filtering, in particular by moving averaging, preferably by forming a weighted moving average, or by a digital filter with a higher order than 1, preferably 2, particularly preferably with a fourth-order digital filter, wherein the control device (18) is preferably further configured to determine the change values on the basis of the smoothed measurement signals.

**13.** Ventilator according to one of the preceding claims,
**characterized in that** the proximal (44) and the distal flow sensor (48) each provide one, preferably exactly one, measured value per breath, which represents the gas flow associated with this breath.

**14.** Ventilator according to one of the preceding claims,
**characterized in that** the proximal flow sensor (44) is a differential pressure sensor.

**15.** Ventilator according to one of the preceding claims,
**characterized in that** the control device (18) is adapted to emit an alarm when it detects an error, and preferably continues to process measurement signals from the proximal flow sensor (44).

**Revendications**

**1.** Appareil respiratoire (10) pour la respiration artificielle avec

- une source de gaz respiratoire (16),
- un agencement de tuyau de ventilation (30) s'étendant entre la source de gaz respiratoire (16) et une extrémité proximale côté patient,
- un dispositif de valve (20, 22) comprenant une valve d'inspiration (20) et une valve d'expiration (22),
- un dispositif de capteur de débit (44, 48) pour la détection quantitative d'un débit de gaz dans le dispositif de tuyau respiratoire (30), comprenant un capteur de débit distal (48) disposé plus loin de l'extrémité côté patient de l'agencement de tuyau de ventilation (30) et un capteur de débit proximal (44) situé plus près de l'extrémité côté patient de l'agencement de tuyau de ventilation (30), et avec
- un dispositif de commande (18) au moins pour le traitement des signaux de mesure de l'agencement de capteur de débit (44, 48), dans lequel le dispositif de commande (18) est adapté pour conclure à une erreur sur la base des signaux de mesure du capteur distal (48) et/ou du capteur proximal (44),

dans lequel le dispositif de commande (18) est adapté pour conclure à une erreur de l'agencement de capteur de débit (44, 48) sur la base d'une comparaison d'une valeur de changement (62, 76) d'un signal de mesure (54, 58, 68, 72) d'un capteur de débit (44) parmi le capteur de débit proximal (44) et le capteur de débit distal (48) avec

- une valeur de changement (60, 74) d'un signal de mesure (52, 56, 66, 70) de l'autre capteur de débit (48) respectif
et/ou avec
- un signal de mesure (54, 58, 68, 72) dudit capteur de débit (44),

**caractérisé en ce que** le dispositif de commande (18) est adapté pour conclure à une erreur de l'agencement de capteur de débit (44, 48) lorsque

- un rapport d'une valeur de somme de valeurs de changement successives (62, 76) du signal de mesure (54, 58, 68, 72) du capteur de débit proximal (44) à une première valeur de référence est supérieur à une valeur de seuil de référence prédéterminée et
- lorsqu'un rapport d'une valeur de somme de valeurs de changement successives (60, 74) du signal de mesure (52, 56, 66, 70) du capteur de débit distal (48) à une deuxième valeur de référence est inférieur à une valeur de seuil de référence prédéterminée.

**2.** Appareil respiratoire selon la revendication 1,
**caractérisé en ce que** le dispositif de commande (18) est adapté pour conclure à une erreur de l'agencement de capteur de débit (44, 48) lorsque la valeur de changement (62, 76 ou 60, 74) du signal de mesure (54, 58, 68, 72 ou 52, 56, 66, 70) d'un capteur de débit (44 ou 48) parmi le capteur de débit distal (48) et le capteur de débit proximal (44) a un sens de changement opposé à la valeur de changement (60, 74 ou 62, 76 60, 74) de l'autre capteur de débit respectif (48 ou 44).

**3.** Appareil respiratoire selon l'une des revendications précédentes,
**caractérisé en ce que** les valeurs de changement (62, 76) du signal de mesure (54, 58, 68, 72) du capteur de débit proximal (44) se suivent directement et/ou que les valeurs de changement (60, 74) du signal de mesure (52, 56, 66, 70) du capteur de débit distal (48) se suivent directement.

**4.** Appareil respiratoire selon l'une des revendications précédentes,
**caractérisé en ce que** la première et/ou la deuxième valeur de référence est une valeur de signal de mesure du capteur de débit proximal (44), en particulier une valeur de signal de mesure du capteur de débit proximal (44) au début ou immédiatement avant le début de la sommation des valeurs de changement (62, 76, 60, 74).

**5.** Appareil respiratoire selon l'une des revendications précédentes,

**caractérisé en ce que** le dispositif de commande (18) est adapté pour commencer la sommation de valeurs de changement (62, 76, 60, 74) lorsque la valeur de signal de mesure et/ou la valeur de changement du capteur de débit proximal (44) est supérieur à une valeur seuil de signal prédéterminée (64).

6. Appareil respiratoire selon la revendication 5,
   **caractérisé en ce que** le dispositif de commande (18) est adapté pour traiter les signaux de mesure du capteur de débit proximal (44) et du capteur de débit distal (48) sur la base d'une phase respiratoire, dans lequel, de préférence lorsque la valeur de signal de mesure du capteur de débit proximal (44) est supérieur à la valeur de signal seuil prédéterminée (64) pendant une phase respiratoire seuil, la première ou/et la deuxième valeur de référence est la valeur de signal de mesure du capteur de débit proximal (48) associé à la phase respiratoire précédant immédiatement la phase respiratoire seuil.

7. Appareil respiratoire selon l'une des revendications précédentes,
   **caractérisé en ce que** le dispositif de commande (18) est adapté pour conclure à une erreur de l'agencement de capteur de débit (44, 48) lorsque

   - un rapport d'une valeur de somme de valeurs de changement du signal de mesure du capteur de débit proximal (44) se produisant successivement, qui de préférence se suivent immédiatement, à une troisième variable de référence est supérieur à une valeur seuil de référence prédéterminée et lorsque
   - un rapport d'une valeur de somme de valeurs de changement du signal de mesure du capteur de débit distal (48) se produisant successivement, qui de préférence se suivent immédiatement, à une quatrième variable de référence est inférieur à une valeur seuil de référence prédéterminée,

   ou pour conclure à une erreur de l'agencement de capteur de débit (44, 48), lorsque

   - un rapport d'une valeur de somme de valeurs de changement du signal de mesure du capteur de débit proximal (44) se produisant successivement, qui de préférence se suivent immédiatement, à une cinquième variable de référence est inférieur à une valeur seuil de référence, et
   - lorsqu'un rapport d'une valeur de somme de valeurs de changement du signal de mesure du capteur de débit distal (48) se produisant successivement, qui de préférence se suivent immédiatement, à une sixième variable de référence est supérieur à une valeur seuil de référence prédéterminée.

8. Appareil respiratoire selon la revendication 7,
   **caractérisé en ce que** le dispositif de commande (18) est adapté pour ne conclure à une erreur de l'agencement de capteur de débit (44, 48) que lorsque en outre

   - la valeur de somme de valeurs de changement du signal de mesure du capteur de débit proximal (44) se produisant successivement, qui de préférence se suivent immédiatement, est supérieur à une première valeur de somme seuil prédéterminée,
   ou/et
   - la valeur de somme de valeurs de changement du signal de mesure du capteur de débit distal (48) se produisant successivement, qui de préférence se suivent immédiatement, est supérieur à une deuxième valeur de somme seuil prédéterminée.

9. Appareil respiratoire selon la revendication 7 ou 8,
   **caractérisé en ce que** la troisième et/ou la cinquième valeur de référence est une valeur de signal de mesure du capteur de débit proximal (44), en particulier une valeur de signal de mesure du capteur de débit proximal (44) au début ou immédiatement avant le début de la sommation des valeurs de changement.

10. Appareil respiratoire selon l'une des revendications 7 à 9,
    **caractérisé en ce que** la quatrième et/ou la sixième valeur de référence est une valeur de signal de mesure du capteur de débit distal (48), en particulier une valeur de signal de mesure du capteur de débit distal (48) au début ou immédiatement avant le début de la sommation des valeurs de changement.

11. Appareil respiratoire selon l'une des revendications 7 à 10,
    **caractérisé en ce que** la sommation des valeurs de changement pour former des valeurs de somme commence lorsque le montant de la valeur de changement du signal de mesure du capteur de débit proximal (44) et/ou distal (48) est supérieur à une valeur seuil de montant prédéterminée (78).

**12.** Appareil respiratoire selon l'une des revendications précédentes, **caractérisé en ce que** le dispositif de commande (18) est adapté pour égaliser les signaux de mesure des capteurs de débit (44, 48) par filtrage, en particulier par calcul de moyenne glissante, de préférence en formant une moyenne pondérée glissante, ou par un filtre numérique d'un ordre supérieur à 1, de préférence à 2, en particulier de préférence avec un filtre numérique du quatrième ordre, le dispositif de commande (18) étant en outre de préférence adapté pour déterminer les valeurs de changement sur la base des signaux de mesure égalisés.

**13.** Appareil respiratoire selon l'une des revendications précédentes, **caractérisé en ce que** le capteur de débit proximal (44) et le capteur de débit distal (48) délivrent chacun une, de préférence exactement une, valeur mesurée par phase respiratoire, qui représente le débit de gaz associé à cette phase respiratoire.

**14.** Appareil respiratoire selon l'une des revendications précédentes, **caractérisé en ce que** le capteur de débit proximal (44) est un capteur de pression différentielle.

**15.** Appareil respiratoire selon l'une des revendications précédentes, **caractérisé en ce que** le dispositif de commande (18) est adapté pour émettre une alarme lorsqu'il conclut à une erreur et de préférence pour continuer à traiter les signaux de mesure du capteur de débit proximal (44).

**Fig. 1**

EP 3 344 317 B1

Fig. 2

Fig. 3

Fig. 4

Fig. 5

EP 3 344 317 B1

Fig. 6

Fig. 7

a)

b)

> 1.08·VTProx

< 0.92·VTServ

**IN DER BESCHREIBUNG AUFGEFÜHRTE DOKUMENTE**

**In der Beschreibung aufgeführte Patentdokumente**

- WO 03055552 A1
- DE 102010040287 A1